# EUROPEAN PATENT APPLICATION

(11) **EP 4 628 121 A1**
(43) Date of publication of application: **08.10.2025**
(21) Application number: 25166519.6
(22) Date of filing: 27.03.2025
(51) Int. Cl.: A61M 1/36, A61M 1/38

(54) **DYNAMIC ADJUSTMENT OF LIGHT INTENSITY AND/OR SIGNAL AMPLIFICATION IN A CENTRIFUGE OPTICAL SENSOR ASSEMBLY**

(30) Priority: 28.03.2024 US 202463571224 P
(71) Applicant: Fenwal, Inc., Lake Zurich, IL 60047 (US)
(72) Inventor: MAHER, Jeffrey R., Lake Zurich, 60047 (US); QUEZADA, Francesca S., Lake Zurich, 60047 (US); DICOLA, Nicholas R., Lake Zurich, 60047 (US)
(74) Representative: Maikowski & Ninnemann Patentanwälte Partnerschaft mbB

(57) **Abstract**

An optical sensor assembly of a centrifuge of a biological fluid separation system includes a light source configured to emit light having an intensity toward a separation chamber received within the centrifuge, with at least a portion of the light exiting the separation chamber as transmitted light. A light detector receives at least a portion of the transmitted light as received light and transmits a signal based on the received light. A controller receives the signal from the light detector, then determines the location of an interface between two of the separated components within the separation chamber based at least in part of the signal. The controller is programmed to determine whether to control the light source to dynamically adjust the intensity of the light during a biological fluid separation procedure and/or to control the light detector to dynamically adjust an amplification of the signal during the procedure.

## Description

### BACKGROUND OF THE INVENTION

### Field of the Disclosure

The present subject matter relates to systems and methods for centrifugally separating biological fluid. More particularly, the present subject matter relates to dynamic adjustment of light intensity and/or signal amplification of an optical sensor assembly of a centrifuge during biological fluid separation procedures.

### Description of Related Art

Various blood processing systems make it possible to collect particular blood constituents, rather than whole blood from a blood source, such as a human donor or patient. Typically, in such systems, whole blood is drawn from a blood source, the particular blood component or constituent is separated, removed and collected, and the remaining blood constituents are returned to the blood source. Removing only particular constituents is advantageous when the blood source is a donor, because potentially less time is needed for the donor's body to return to normal or pre-donation levels. Also, donations of particular blood components or constituents may be made at more frequent intervals than when whole blood is collected. This increases the overall supply of blood constituents, such as plasma and platelets, made available for transfer and/or therapeutic treatment or health care.

Whole blood typically may be separated into its constituents through centrifugation. This requires that the whole blood be passed through a centrifuge after it is withdrawn from, and before it is returned to, the blood source. To reduce contamination and possible infection, if the blood source is a donor or patient, the blood preferably is contained and processed within a disposable, sealed, sterile fluid flow circuit during the entire centrifugation process. Disposable flow circuits include a separation chamber portion, which an operator installs in a durable, reusable centrifuge assembly containing reusable hardware (centrifuge, drive system, pumps, valve actuators, programmable controller, and the like) that rotates the separation chamber and controls the flow through the disposable flow circuit during its use when mounted on and in cooperation with the hardware. The centrifuge assembly engages and rotates the separation chamber of the fluid flow circuit during a separation procedure. The blood, however, makes actual contact only with the fluid flow circuit, which is used only once and then discarded.

Prior to or shortly after loading a disposable circuit into the centrifuge assembly, the operator typically enters, for example, by means of a touch screen or other user interface system, a particular processing protocol to be executed by the system (e.g., a procedure wherein platelets are separated from whole blood and collected) and other parameters (e.g., the weight of the donor, the desired volume of the separated blood component to be collected, etc.). When the system has been programmed, the operator phlebotomizes a donor and the system carries out the procedure, under the supervision of the operator.

As the centrifuge assembly rotates the separation chamber of the disposable flow circuit, the heavier (greater specific gravity) components of the whole blood in the separation chamber, such as red blood cells, move radially outwardly away from the center of rotation toward the outer or "high-G" wall of the separation chamber. The lighter (lower specific gravity) components, such as plasma, migrate toward the inner or "low-G" wall of the separation chamber. Various components can be selectively removed from the whole blood by including appropriately located channeling structures and outlet ports in the separation chamber of the disposable flow circuit. For example, therapeutic plasma exchange involves separating plasma from cellular blood components, collecting the plasma, and returning the cellular blood components and a replacement fluid to the blood source. Alternatively, red blood cells may be harvested from the separation chamber and the rest of the blood constituents returned to the donor. Other processes are also possible including, without limitation, platelet collection, red blood cell exchanges, plasma exchanges, etc. or a combination of the above collections.

Optimal separation requires that the interface between the separated blood components be located at a target location between the high-G and low-G walls of the separation chamber, which is illustrated in Fig. 1. For example, when performing a therapeutic plasma exchange procedure, the interface between the plasma and the cellular blood components affects the performance of the system. If the interface is located too close to the low-G wall (as shown in Fig. 2), then the collected plasma may become unduly populated or contaminated by cellular blood components. On the other hand, if the interface is located too far from the low-G wall (as shown in Fig. 3), there may be no contamination of the plasma, but the separation efficiency of the system may be decreased with less plasma collected over time.

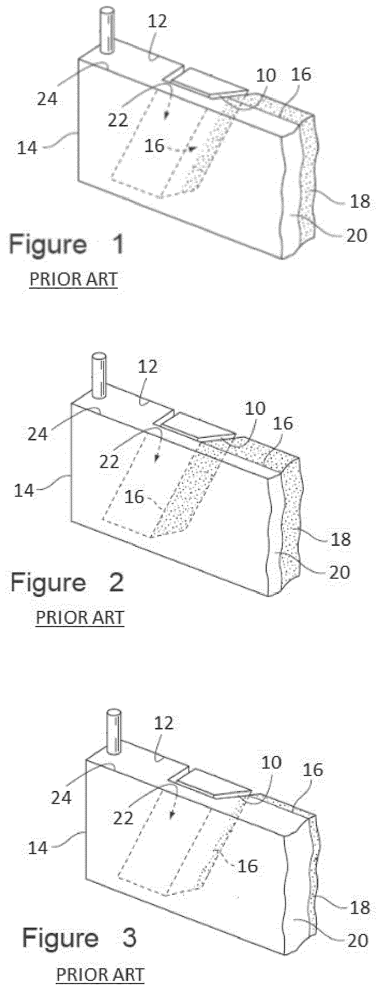

Various centrifuges, such as those shown and described in U.S. Patent Nos. 6,254,784 to Nayak et al.; 10,768,107 to Koudelka et al.; and 11,465,160 to Min et al. (which are incorporated herein by reference), are operable to automatically keep the interface at a target location as the centrifuge operates. In these three systems, a transparent or translucent ramped surface 10 (Figs. 1-3) is associated with the radially outer wall 12 of the separation chamber 14 to allow light from a light source to enter into the separation chamber 14 and encounter a fluid being separated therein or one or more separated fluid components. In the case of blood being separated, the interface 16 between the generally dark, opaque red blood cell layer 18 and the generally light, clear plasma layer 20 appears as a line on the ramped surface 10. The position of the line on the ramped surface 10 is a function of the radial position of the interface 16 between the red blood cells 18 and plasma 20 within a channel 22 defined by the high-G wall 12 and low-G wall 24 of the separation chamber 14. Accordingly, the position of the line on the ramped surface 10 can be used to gauge the position of the interface 16 between the high-G wall 12 and low-G wall 24.

Automatic control over the location of the interface has been achieved by sensing the position of the line on the ramped surface and thereafter adjusting the centrifuge operating parameters to place and keep the line within desired limits. For example, by controlling the rate at which plasma is withdrawn from the separation chamber, the line can be "moved" up (radially inwardly, by increasing the plasma flow rate) or down (radially outwardly, by decreasing the plasma flow rate) on the ramped surface. An optical sensor assembly may be used to sense the position of the line on the ramped surface. Optical control systems commonly operate based on the principle that light will transmit through optically clear fluid, such as saline and/or plasma (which may include platelet-rich plasma or platelet-poor plasma), while light will not transmit through optically dense fluid, such as whole blood or packed red blood cells. Thus, when using a light source and detector apparatus, as in the prior systems, optical signals representative of the optical clear fluid thickness within a centrifuge can be measured and applied to determine, correct, and maintain the location of the red blood cell/plasma interface.

More particularly, as the centrifuge spins the separation chamber, the ramped surface will rotate into and then out of the path of light emitted by a light source. When an optically transparent or translucent fluid (e.g., saline or plasma) is aligned with the light source, the light will be transmitted through the fluid and be received by a light detector. While the light detector is receiving light from the light source, it will generate a signal having a voltage that corresponds to the intensity of light received by the light detector, with the signal being received by a controller. At all other times (e.g., when the ramped surface is out of alignment with the light source and when the light encounters an optically opaque fluid, such as red blood cells, on the ramped surface), light will not be transmitted to the light detector, with the light detector either sending no signal to the controller or a "low" signal having a voltage that is significantly lower than the voltage of the signal that is generated when light is being received by the light detector.

The light detector will, thus, receive an elevated amount of light for a certain amount of time during each complete rotation of the centrifuge, with that amount of time corresponding to the amount of time that the optically transparent or translucent fluid on the ramp is aligned with the light source. As such, the signal transmitted by the light detector and received by the controller when the light detector is receiving light has both a magnitude (voltage) and duration, which may be referred to as its "pulse width." As the position of the interface within the channel of the separation chamber moves closer to the high-G wall of the separation chamber (as in Fig. 3), the pulse width of the signal will increase, while the pulse width of the signal will decrease as the interface moves closer to the low-G wall (as in Fig. 2). The controller compares the pulse width of an individual signal to a target pulse width that corresponds to a target location of the interface within the channel of the separation chamber (as in Fig. 1) and makes appropriate adjustments to the operation of other components of the system (e.g., changing the rate at which plasma is withdrawn from the separation chamber) to move the interface toward the target location (with the controller confirming that the interface is at the target location when the measured pulse width is equal to the target pulse width).

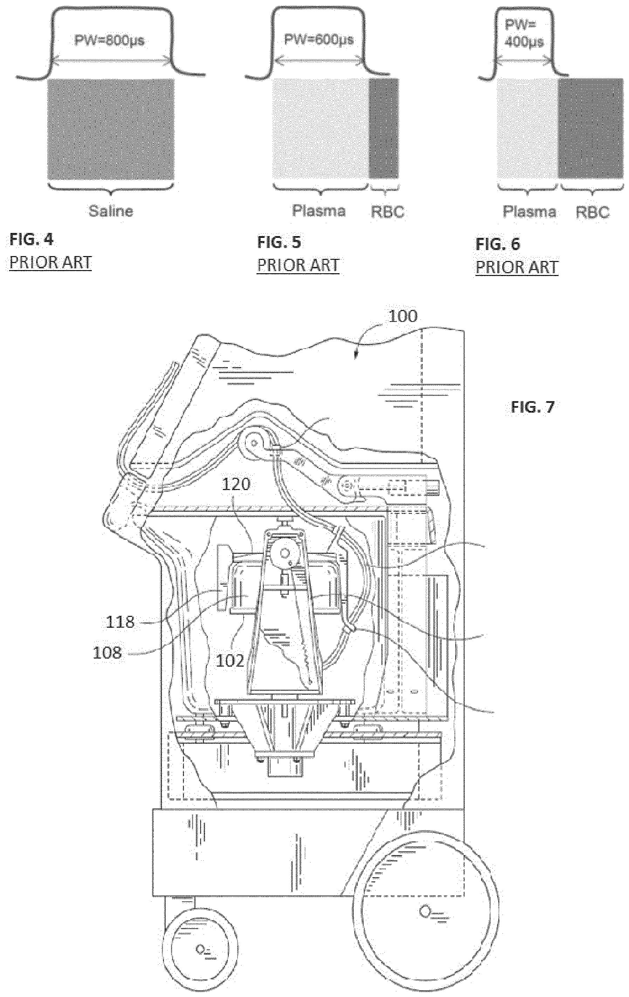

Figs. 4-6 illustrate exemplary signals corresponding to different fluid conditions within a separation chamber. Fig. 4 shows a signal that is generated by the light detector when saline is flowing through the separation chamber, which may occur at the start of a fluid separation procedure, during a priming stage of the procedure. Light from the light source will be transmitted through the saline and received by the light detector over the entire width of the ramped surface, such that the signal generated by the light detector will have a maximum pulse width (which will depend upon the width of the ramped surface and the rotational rate of the centrifuge). This signal, which may be referred to as a "Saline Calibration Signal," may be used by the controller during a fluid separation procedure to calculate the position of the interface, as will be described in greater detail.

Fig. 5 shows a signal that is generated by the light detector when red blood cells ("RBC") occupy a relatively small percentage of the width of the ramped surface. As shown in Fig. 5 and described above, the light detector will only transmit a signal having a relatively high voltage when a light-transmissive fluid (e.g., plasma) on the ramped surface is aligned with the light source. In the situation shown in Fig. 5, plasma occupies only 75% of the width of the ramped surface, such that the resulting signal will have a pulse width that is 75% of the pulse width of the Saline Calibration Signal. In an exemplary embodiment, the target pulse width may be 60% of the pulse width of the Saline Calibration Signal, in which case the controller will take appropriate action to cause plasma to occupy a smaller percentage of the width of the ramped surface until a signal from the light detector is found by the controller to have a pulse width that is 60% of the pulse width of the Saline Calibration Signal (corresponding to the target interface position shown in Fig. 1).

Fig. 6 shows a signal that is generated by the light detector when red blood cells occupy a larger percentage of the width of the ramped surface. As shown in Fig. 6 and described above, the light detector will only transmit a signal having a relatively high voltage when a light-transmissive fluid (e.g., plasma) on the ramped surface is aligned with the light source. In the situation shown in Fig. 6, plasma occupies only 50% of the width of the ramped surface, such that the resulting signal will have a pulse width that is 50% of the pulse width of the Saline Calibration Signal. In the exemplary embodiment in which the target pulse width is 60% of the pulse width of the Saline Calibration Signal, the controller will take appropriate action to cause plasma to occupy a greater percentage of the width of the ramped surface until a signal from the light detector is found by the controller to have a pulse width that is 60% of the pulse width of the Saline Calibration Signal.

While such optical sensor assemblies have proven to be effective, their operation may be impaired by any of a number of possible irregularities in the composition of a fluid to be separated, in the configuration of the separation chamber, and/or in the configuration and/or operation of a component of the centrifuge. The existence of any of these irregularities may result in an inaccurate determination of the interface location, possibly leading to poor performance, separation and/or product collection. By way of example, a condition such as lipemia (in which there is an unusually high concentration of lipids in blood) will decrease the optical clarity of plasma. In certain embodiments of the above-described optical sensor assemblies, the low-G side of the fluid gap defined by the ramped surface is rotated into alignment with the light source before the high-G side, such that the rising edge of the signal will be indicative of the nature of the fluid present at the low-G side of the fluid gap, while the falling edge of the signal will be indicative of the nature of the fluid present at the high-G side. Due to the design of the ramped surface applied in all three systems, the thickness of the fluid (in the radial direction) through which light from the light source travels is greater at the low-G side of the fluid gap than at the high-G side. On account of the light having to traverse a different amount of lipemic plasma at all points along the width of the ramped surface where the plasma is present, a different amount of light will be transmitted through the plasma and received by the light detector during a single rotation of the ramped surface past the light source. This results in a signal having a varying voltage along its pulse width, rather than one having a relatively uniform voltage of the type shown in Figs. 4-6. When the pulse width of the signal is measured at only positions having a voltage that is greater than a minimum percentage of the maximum voltage, the calculated pulse width will be shorter than it would be if the plasma were not lipemic, resulting in the controller determining that the plasma occupies a smaller percentage of the width of the ramped surface than it actually does. This incorrect determination of the position of the interface may lead to the controller making improper adjustments to the operational parameters of the various components of the centrifuge, possibly resulting in the controller causing the interface to be moved to a position that is different from the target position.

Possible hardware and disposable irregularities include a light source that is emitting light having an intensity that is either too high or too low and a ramped surface having sinks or voids or experiencing crazing or cracking. Possible solutions to such issues include replacing the hardware components on a device-by-device basis, sorting light sources to provide the optimum configuration at manufacturing, and overhauling the entire design of the optical sensor assembly to be more robust. None of these options are cost effective or necessarily feasible from a business perspective.

### SUMMARY

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, an optical sensor assembly is provided for a biological fluid separation system including a centrifuge configured to receive a separation chamber in which a biological fluid is separated into at least two separated components. The optical sensor assembly includes a light source, a light detector and a controller. The light source is configured to emit light having a first intensity toward the separation chamber, with at least a portion of the light exiting the separation chamber as transmitted light. The light detector is configured to receive at least a portion of the transmitted light as received light and to transmit a signal having a voltage and a pulse width, with the voltage being based at least in part on a second intensity of the received light. The controller is programmed to receive the signal from the light detector and determine a location of an interface between at least two of the separated components within the separation chamber based at least in part on the signal, with the controller being further programmed to control the light source to dynamically adjust the first intensity during a biological fluid separation procedure and/or to control the light detector to dynamically adjust an amplification of the signal during the biological fluid separation procedure.

### BRIEF DESCRIPTION OF DRAWINGS

Fig. 1 is an enlarged perspective view of a portion of a centrifugal separation chamber according to a conventional design, showing the centrifugally separated red blood cell layer, plasma layer, and interface within the chamber when in a target location on a ramped surface;
Fig. 2 is an enlarged perspective view of the separation chamber of Fig. 1, showing the interface within the chamber spaced away from the target location, adjacent to a low-G wall of the chamber;
Fig. 3 is an enlarged perspective view of the separation chamber shown in Fig. 1, showing the interface within the chamber spaced away from the target location, adjacent to a high-G wall of the chamber.
Fig. 4 is a schematic diagram of an optical signal generated by a conventional light detector when monitoring a centrifugal separation chamber filled with saline;
Figs. 5 and 6 are schematic diagrams of optical signals generated by a conventional light detector when monitoring a centrifugal separation chamber filled with a biological fluid that has been separated into two components;
Fig. 7 is a side elevation view, with portions broken away and in section, of a biological fluid separation system employing aspects of the present disclosure, with a centrifuge bowl and spool of the system being shown in their operating position;

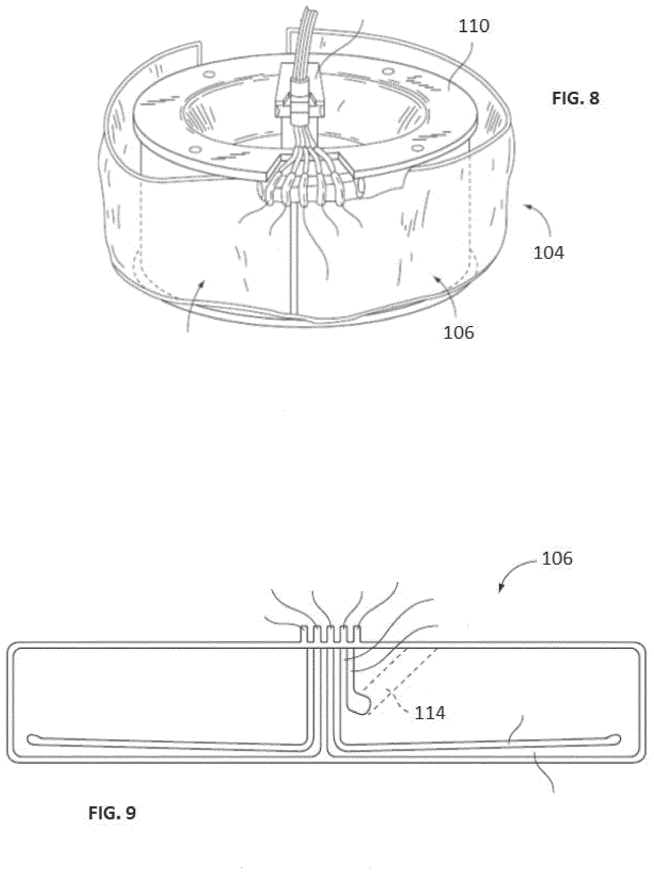

Fig. 8 is a top perspective view of the spool of the centrifuge shown in Figure 7 carrying a separation chamber;
Fig. 9 is a plan view of the separation chamber shown in Fig. 8, out of association with the spool;

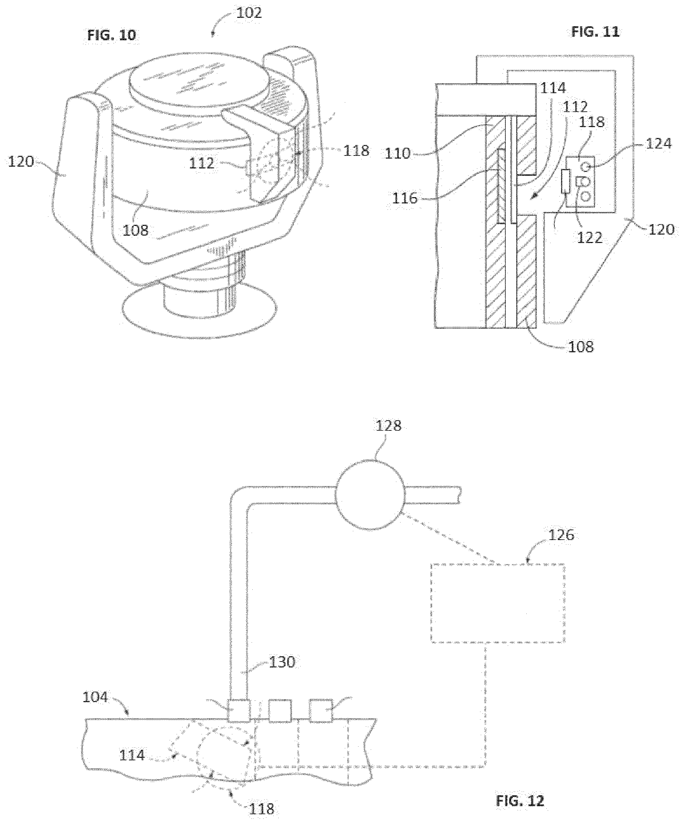

Fig. 10 is a side perspective view of the bowl and spool of the centrifuge of Fig. 7, showing an optical sensor assembly being carried by a yoke of the centrifuge to monitor fluid separation within the centrifuge;
Fig. 11 is a side section view of the bowl, spool, and optical sensor assembly when the viewing head is aligned with a ramped surface of the bowl;
Fig. 12 is a schematic view of selected components of the optical sensor assembly, along with a pump controlled by a controller of the optical sensor assembly;

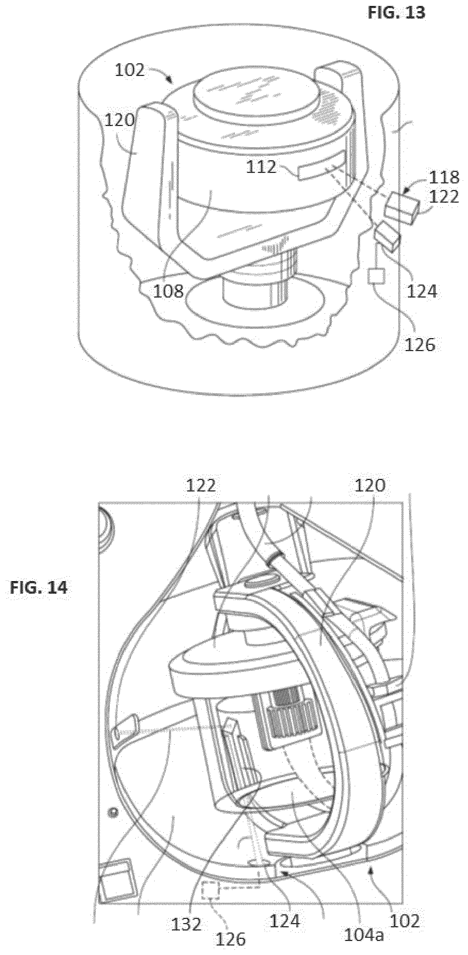

Fig. 13 a perspective view of another embodiment of an exemplary centrifuge according to an aspect of the present disclosure, with portions of a centrifuge bucket broken away for illustrative purpose;
Fig. 14 is a perspective view of yet another embodiment of an exemplary centrifuge according to an aspect of the present disclosure; and

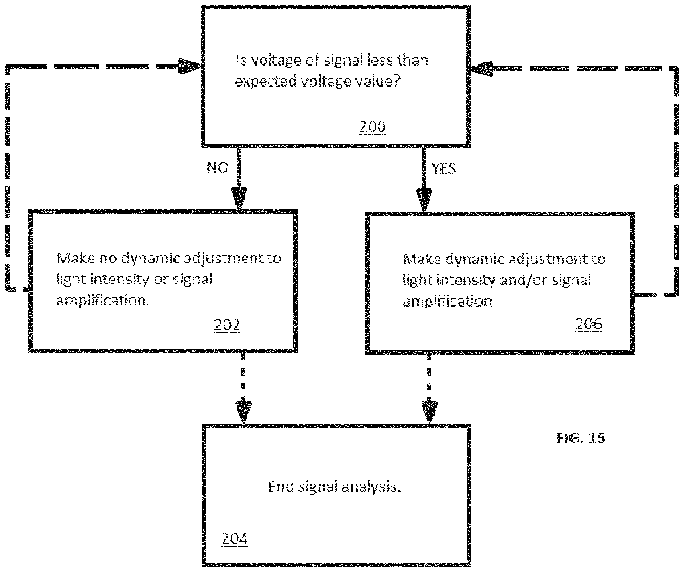

Figs. 15-20 are flow charts of exemplary embodiments of an approach to determining whether a dynamic adjustment to light intensity and/or signal amplification of an optical sensor assembly is required, according to an aspect of the present disclosure.

### DETAILED DESCRIPTION

The embodiments disclosed herein are for the purpose of providing a description of the present subject matter, and it is understood that the subject matter may be embodied in various other forms and combinations not shown in detail. Therefore, specific designs and features disclosed herein are not to be interpreted as limiting the subject matter as defined in the accompanying claims.

Optical sensor assemblies and optical interface monitoring techniques according to the present disclosure will be described herein in the context of a biological fluid separation system employing a ramped surface of the type described above. However, it should be understood that differently configured biological fluid separation systems (including those omitting a ramped surface as part of an interface detection assembly) may be employed in combination with the optical sensor assemblies and techniques described herein.

Figs. 7-12 show one embodiment of a biological fluid separation system 100 embodying aspects of the present disclosure. The biological fluid separation system 100 is configured generally in accordance with the system described in U.S. Patent No. 6,254,784 and generally in accordance with the configuration of the AMICUS^{®} separator marketed by Fenwal, Inc. of Lake Zurich, Illinois, which is an affiliate of Fresenius Kabi AG of Bad Homburg, Germany.

In short, the biological fluid separation system 100 includes a centrifuge 102 configured to receive a separation chamber 104 of a disposable fluid flow circuit 106 (Fig. 8), with the separation chamber 104 being removably positioned in a generally annular gap between an outer bowl 108 and an inner spool 110. The bowl 108 includes an opening or window 112 having an associated ramped surface 114, with the spool 110 including a mirror or reflector 116 aligned with the ramped surface 114. Fig. 9 illustrates the position of the ramped surface 114 with respect to the separation chamber 104 when the separation chamber 104 is mounted within the centrifuge 102.

Selected components of an optical sensor assembly 118 (Figs. 10 and 11) are mounted to a portion of the centrifuge 102 that rotates during a biological fluid separation procedure (e.g., being associated to a yoke 120 of the centrifuge 102). The optical sensor assembly 118 includes a light source 122 and a light detector 124, with the light detector 124 being electrically coupled to a controller 126 (Fig. 12). The light source 122 (which may be configured as a laser or as one or more light-emitting diodes, for example) emits a light that passes through the ramped surface 114 and fluid aligned with the ramped surface 114 (as described above), with the mirror or reflector 116 reflecting light that has been transmitted through the fluid back through the separation chamber 104 and to the light detector 124 (which may be configured, for example, as a PIN diode detector). In accordance with the above description, the light detector 124 generates a signal based on the intensity of the reflected light that it has received, with the signal being transmitted to the controller 126, which determines the position of the interface between separated fluid components within the separation chamber 104 and takes appropriate action to cause the interface to be moved toward a target location. In the embodiment shown in Fig. 12, the controller 126 commands a pump 128 associated with a plasma outlet line 130 of the fluid flow circuit 106 to adjust the rate at which plasma is removed from the separation chamber 104 so as to cause the interface to move in the desired direction, but the controller 126 may be programmed to take any other corrective action without departing from the scope of the present disclosure.

Reference may be made to U.S. Patent No. 6,254,784 for additional details regarding the configurations of the biological fluid separation system 100 and the fluid flow circuit 106 and the manner in which the two cooperate to execute a biological fluid separation procedure.

Figs. 13 and 14 illustrate two alternative embodiments of the biological fluid separation system 100 of Figs. 7-12. The embodiment of Fig. 13 is configured generally in accordance with the system described in U.S. Patent No. 10,768,107 and generally in accordance with the configuration of the AMICORE^{®} separator marketed by Fenwal, Inc. Compared to the biological fluid separation system 100 of Figs. 7-12, the device of Fig. 13 differs primarily to the extent that its optical sensor assembly 118 is mounted to a component of the centrifuge 102 that is stationary during a biological fluid separation procedure (e.g., the enclosure or "bucket" of the centrifuge 102), rather than being mounted to a component that is rotated during such a procedure. The optical sensor assembly 118 otherwise is configured and operates in accordance with the above description.

As for the embodiment of Fig. 14, it is configured generally in accordance with the system described in U.S. Patent No. 11,465,160. The embodiment of Fig. 14 is similar to the embodiment of Fig. 13, with components of an optical sensor assembly that are configured to be stationary during a biological fluid separation procedure, though the embodiment of Fig. 14 employs a light source 122 that is spaced apart from the light detector 124, rather than being positioned generally adjacent thereto. More particularly, the separation chamber 104a of Fig. 14 is provided with a prismatic reflector 132, which receives light from the light source 122 (after the light has passed through fluid within the separation chamber 104a) and directs the light along a path that is generally perpendicular to the initial path of the light, with at least a portion of the redirected light being received by the light detector 124.

Additionally, whereas the embodiments of Figs. 7-13 employ a separation chamber 104 that is formed of a relatively flexible material and used in combination with a ramped surface 114 that is incorporated into a component of the centrifuge, the separation chamber 104a is instead formed of a rigid material, with a ramped surface being incorporated into the separation chamber 104a, in alignment with the prismatic reflector 132. The separation chamber 104a may be formed of a transparent or translucent material, in which case the light from the light source 122 will always be passing through the outer wall of the separation chamber 104a and striking the fluid within the separation chamber 104a. However, the light from the light source 122 will only be directed to the light detector 124 when the light has passed through the ramped surface and through a light-transmissive fluid (e.g., plasma) aligned with the ramped surface and reaches the prismatic reflector 132. It will, thus, be seen that the ramped surface and prismatic reflector 132 perform a similar function to the ramped surface and mirror or reflector of the embodiments of Figs. 7-13 to direct light to the light detector 124 in order to develop a signal having a voltage and pulse width, as described above.

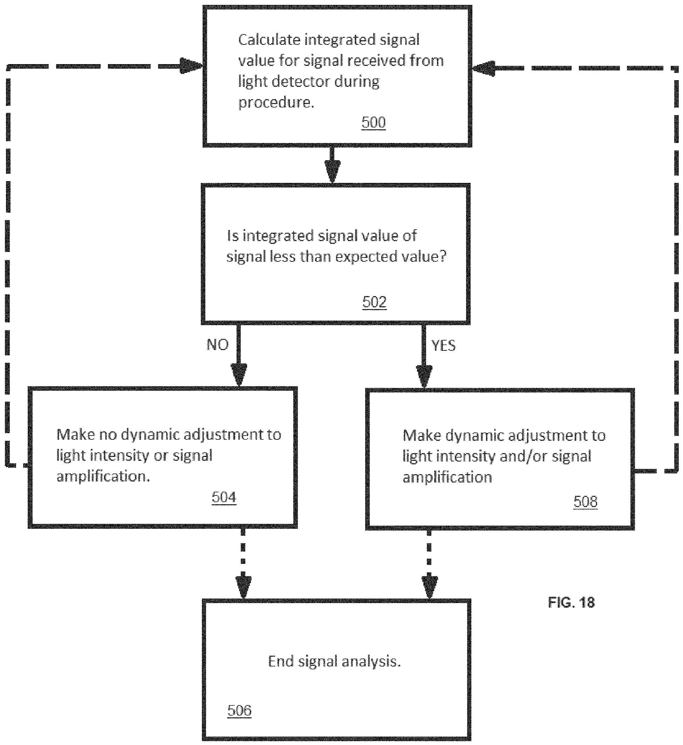

Regardless of the particular configuration of the optical sensor assembly, the controller 126 is programmed so as to be able to dynamically adjust the intensity of the light emitted by the light source 122 and/or the amplification of the signal that is transmitted from the light detector 124 to the controller 126 during a biological fluid separation procedure. Figs. 15-20 illustrate exemplary algorithms that may be executed by the controller 126 during a biological fluid separation procedure to determine whether to adjust the intensity of the light emitted by the light source 122 and/or the amplification of the signal that is transmitted from the light detector 124 to the controller 126. Figs. 15-17 illustrate approaches in which the controller 126 analyzes the voltage of a signal that it has received from the light source 122, while Figs. 18-20 illustrate approaches in which the controller 126 calculates and analyzes an integrated signal value, which employs both the voltage of the signal and the pulse width.

It should be understood that the illustrated algorithms are merely exemplary and that they may be modified without departing from the scope of the present disclosure. For example, while Figs. 15-20 refer to the voltage of a "signal" or the integrated signal value for a "signal," any of the algorithms may include an initial sampling step and/or a preliminary step in which an average is calculated in order to provide a "signal" to be analyzed. In one exemplary embodiment, this may include the controller 126 determining the median voltage across a portion of a signal or of the entire recorded pulse widths by sampling and averaging (e.g., by sorting the set of voltage values) prior to determining any necessary adjustment. The result of any such initial or preliminary steps is then treated as a "signal" to be analyzed using an algorithm or approach according to the present disclosure. As appropriate, the algorithm may include further steps to identify and possibly exclude outliers from the dataset to ensure the integrity of the "signal" that is obtained using any initial or preliminary steps.

In a first step 200 of the procedure of Fig. 15, the controller 126 compares the voltage of a signal from the light detector 124 to an "expected" voltage value. As explained, the "signal" and its voltage may be the result of one or more initial or preliminary steps involving sampling and/or averaging, with the voltage compared to the expected value being, for example, a maximum voltage of the signal that is recorded during one or more subsequent pulse widths, the median voltage across a portion of the signal or of the entire recorded pulse widths, or an average voltage of the signal during the pulse widths (with any average values being calculated according to any suitable approach without departing from the scope of the present disclosure). The expected value may be pre-programmed into the controller 126 (which may include being provided to the controller 126 by an operator at the beginning of a biological fluid separation procedure) or determined by the controller 126. For example, the controller 126 may select an expected value that is based on the voltage of a reference or calibration signal received by the controller 126 during a priming or calibration stage of the procedure, such as the above-described "Saline Calibration Signal." The expected value may be equal to the voltage of the reference or calibration signal or to a predetermined percentage of the voltage of such a signal (e.g., with the expected value being set to 75% of the voltage of the reference or calibration signal).

The next step of the procedure depends on the comparison executed in step 200. When the voltage of the signal received by the controller 126 is greater than or equal to the expected voltage value, the controller 126 proceeds to step 202 in which it has determined that there is no need for an adjustment to either the intensity of the light emitted by the light source 122 or the amplification of the signal emitted by the light detector 124 and makes no such adjustment. From there, the controller 126 may either return to step 200 (if the process is to be repeated for a subsequent signal) or may proceed to step 204 in which the signal assessment procedure is ended (if the controller 126 is programmed to check only once during a biological fluid separation procedure whether a dynamic adjustment is required or is at least programmed to not automatically repeat the procedure of Fig. 15 for successive signals).

On the other hand, when the voltage of the signal received by the controller 126 is less than the expected voltage value, the controller 126 proceeds to step 206 in which it has determined that a dynamic adjustment (increment) of the intensity of light emitted by the light source 122 and/or the amplification of the signal emitted by the light detector 124 is required and implements such an adjustment. The exact adjustment that is implemented by the controller 126 in step 206 may take any of a variety of possible forms. For example, the controller 126 may only command the light source 122 to emit light having a greater intensity in step 206, with the magnitude of the change being based on a difference between the voltage values compared in step 200. In another embodiment, the controller 126 may only command the light detector 124 (which may include commanding an amplification component or module of the light detector 124) to increase the amplification of the signals being generated by the light detector 124, again with the magnitude of the change being based on a difference between the voltage values compared in step 200. In yet another embodiment, the controller 126 may command both the light source 122 to emit light having a greater intensity and the light detector 124 to increase the amplification of the signals being generated by the light detector 124, with both changes being informed by the difference between the voltage values compared in step 200.

After making a dynamic adjustment in step 206, the controller 126 may either return to step 200 (if the process is to be repeated for a subsequent signal) or may proceed to step 204 in which the signal assessment procedure is ended (if the controller 126 is programmed to check only once during a biological fluid separation procedure whether a dynamic adjustment is required or is at least programmed to not automatically repeat the procedure of Fig. 15 for successive signals)

In one embodiment, the controller 126 may be programmed to first adjust the signal amplification in step 206 before adjusting the light intensity, based on the presumption that the light source 122 is operating properly and that a low-voltage signal is due to an irregularity in the biological fluid (e.g., if separated plasma is lipemic). If the controller 126 then repeats the process of Fig. 15 for a subsequent signal and finds in step 200 that the adjustment to the signal amplification has not been effective to increase the voltage of the subsequent signal to at least equal the expected value, it may proceed to command the light source 122 to increase the light intensity in step 206 to see whether such an adjustment is more effective in increasing the voltage of later signals.

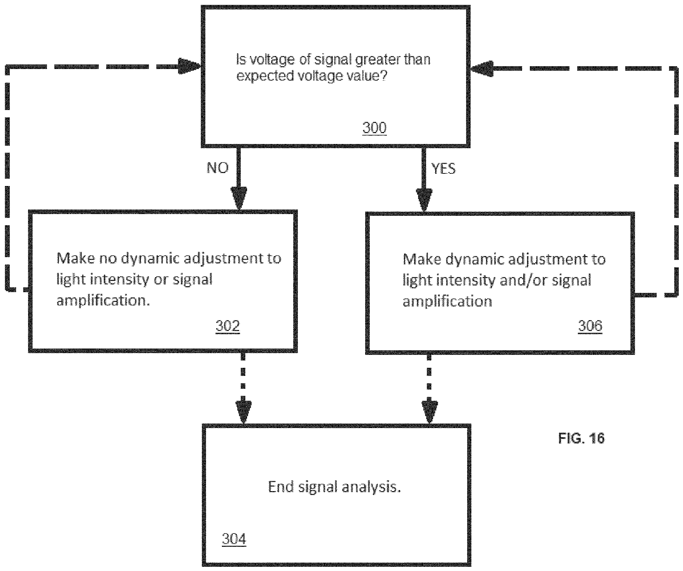

Fig. 16 illustrates a variation of the algorithm of Fig. 15, with the controller 126 determining in step 300 whether the voltage of a signal is greater than an expected value, rather than determining whether the voltage is less than an expected value. When the voltage of the signal received by the controller 126 is less than or equal to the expected voltage value, the controller 126 proceeds to step 302 in which it has determined that there is no need for an adjustment to either the intensity of the light emitted by the light source 122 or the amplification of the signal emitted by the light detector 124 and makes no such adjustment. From there, the controller 126 may either return to step 300 (if the process is to be repeated for a subsequent signal) or may proceed to step 304 in which the signal assessment procedure is ended (if the controller 126 is programmed to check only once during a biological fluid separation procedure whether a dynamic adjustment is required or is at least programmed to not automatically repeat the procedure of Fig. 16 for successive signals).

On the other hand, when the voltage of the signal received by the controller 126 is greater than the expected voltage value, the controller 126 proceeds to step 306 in which it has determined that a dynamic adjustment (decrement) of the intensity of light emitted by the light source 122 and/or the amplification of the signal emitted by the light detector 124 is required and implements such an adjustment. The exact adjustment that is implemented by the controller 126 in step 306 may take any of a variety of possible forms. For example, the controller 126 may only command the light source 122 to emit light having a lesser intensity in step 306, with the magnitude of the change being based on a difference between the voltage values compared in step 300. In another embodiment, the controller 126 may only command the light detector 124 (which may include commanding an amplification component or module of the light detector 124) to decrease the amplification of the signals being generated by the light detector 124, again with the magnitude of the change being based on a difference between the voltage values compared in step 300. In yet another embodiment, the controller 126 may command both the light source 122 to emit light having a lesser intensity and the light detector 124 to decrease the amplification of the signals being generated by the light detector 124, with both changes being informed by the difference between the voltage values compared in step 300.

After making a dynamic adjustment in step 306, the controller 126 may either return to step 300 (if the process is to be repeated for a subsequent signal) or may proceed to step 304 in which the signal assessment procedure is ended (if the controller 126 is programmed to check only once during a biological fluid separation procedure whether a dynamic adjustment is required or is at least programmed to not automatically repeat the procedure of Fig. 16 for successive signals).

In one embodiment, the controller 126 may be programmed to first adjust the signal amplification in step 306 before adjusting the light intensity, based on the presumption that the light source 122 is operating properly and that a high-voltage signal is due to variations in the signal compared to the baseline (e.g., contamination of a platelet product with white and/or red blood cells). If the controller 126 then repeats the process of Fig. 16 for a subsequent signal and finds in step 300 that the adjustment to the signal amplification has not been effective to decrease the voltage of the subsequent signal to at least equal the expected value, it may proceed to command the light source 122 to decrease the light intensity in step 306 to see whether such an adjustment is more effective in decreasing the voltage of later signals.

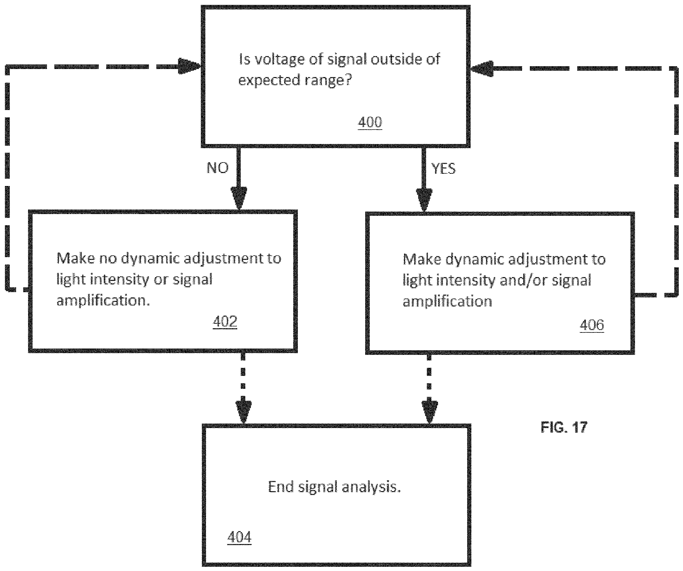

The procedure of Fig. 17 is similar to the procedures of Figs. 15 and 16, but compares the voltage of one or more signals to an "expected" voltage range (per step 400), rather than comparing the voltage of the signal(s) to a single expected voltage value (as in step 200 of Fig. 15 and step 300 of Fig. 16). As explained, the voltage compared to the expected range may be the result of one or more initial or preliminary steps, with the voltage being, for example, the median voltage across a portion of the signal or of the entire recorded pulse widths, or an average voltage of the signal during the pulse widths. The expected value range may be pre-programmed into the controller 126 (which may include being provided to the controller 126 by an operator at the beginning of a biological fluid separation procedure) or determined by the controller 126. For example, the controller 126 may select an expected value range that is based on the voltage of a reference or calibration signal received by the controller 126 during a priming or calibration stage of the procedure, such as the above-described "Saline Calibration Signal." The expected value range may be based on selected percentages of the voltage of the reference or calibration signal (e.g., with the expected voltage range being set to 75 - 95% of the voltage of the reference or calibration signal).

The next step of the procedure depends on the comparison executed in step 400. When the voltage of the signal received by the controller 126 is within the expected voltage range, the controller 126 proceeds to step 402 in which it has determined that there is no need for an adjustment to either the intensity of the light emitted by the light source 122 or the amplification of the signal emitted by the light detector 124 and makes no such adjustment. From there, the controller 126 may either return to step 400 (if the process is to be repeated for a subsequent signal) or may proceed to step 404 in which the signal assessment procedure is ended (if the controller 126 is programmed to check only once during a biological fluid separation procedure whether a dynamic adjustment is required or is at least programmed to not automatically repeat the procedure of Fig. 17 for successive signals).

On the other hand, when the voltage of the signal received by the controller 126 is outside of the expected voltage range, the controller 126 proceeds to step 406 in which it has determined that a dynamic adjustment of the intensity of light emitted by the light source 122 and/or the amplification of the signal emitted by the light detector 124 is required and implements such an adjustment. The exact adjustment that is implemented by the controller 126 in step 406 may take any of a variety of possible forms. For example, the controller 126 may only command the light source 122 to emit light having a greater intensity (when the voltage is below the expected voltage range) or a lesser intensity (when the voltage is above the expected voltage range) in step 406, with the magnitude of the change being based on a difference between the voltage values compared in step 400. In another embodiment, the controller 126 may only command the light detector 124 (which may include commanding an amplification component or module of the light detector 124) to increase the amplification of the signals being generated by the light detector 124 (when the voltage is below the expected voltage range) or to decrease the signal amplification (when the voltage is above the expected voltage range), again with the magnitude of the change being based on a difference between the voltage values compared in step 400. In yet another embodiment, the controller 126 may command both the light source 122 to emit light having a different intensity and the light detector 124 to adjust the signal amplification, with both changes being informed by the difference between the voltage values compared in step 400.

After making a dynamic adjustment in step 406, the controller 126 may either return to step 400 (if the process is to be repeated for a subsequent signal) or may proceed to step 404 in which the signal assessment procedure is ended (if the controller 126 is programmed to check only once during a biological fluid separation procedure whether a dynamic adjustment is required or is at least programmed to not automatically repeat the procedure of Fig. 17 for successive signals).

In one embodiment, when the voltage is below the expected voltage range, the controller 126 may be programmed to first increase the signal amplification in step 406 before adjusting the light intensity, based on the presumption that the light source 122 is operating properly and that a low-voltage signal is due to an irregularity in the biological fluid (e.g., if separated plasma is lipemic). If the controller 126 then repeats the process of Fig. 17 for a subsequent signal and finds in step 400 that the adjustment to the signal amplification has not been effective to increase the voltage of the subsequent signal so as to bring it within the expected range, it may proceed to command the light source 122 to increase the light intensity in step 406 to see whether such an adjustment is more effective in increasing the voltage of later signals.

Similarly, when the voltage is above the expected voltage range, the controller may be programmed to first decrease the signal amplification, based on the presumption that the default or initial intensity of the light from the light source 122 should not result in a signal having a voltage that is greater than the expected range. If the controller 126 then repeats the process of Fig. 17 for a subsequent signal and finds in step 400 that the adjustment to the signal amplification has not been effective to decrease the voltage of the subsequent signal so as to bring it within the expected range, it may proceed to command the light source 122 to decrease the light intensity in step 406 to see whether such an adjustment is more effective in decreasing the voltage of later signals.

Turning now to the protocol of Fig. 18, it is similar to the procedure of Fig. 15, but calls for the controller 126 to analyze both the voltage and the pulse width of a signal from the light detector 124, rather than considering only the signal voltage. In a first step 500 of the procedure of Fig. 18, the controller 126 calculates an integrated signal value for a signal from the light detector 124, with the integrated signal value being indicative of the area under a curve representing the signal (with Figs. 4-6 illustrating exemplary signal curves). The integrated signal value may be calculated by multiplying the voltage of the signal by the pulse width (which is an approximate value of the area under the curve representing the signal) or by calculating the integral of the curve or by any other suitable approach. This may include calculating the area of only a portion of the signal curve (e.g., considering only the portion of the curve in which the voltage of the signal is at least a minimum percentage of the maximum voltage). Additionally, the integrated signal value may be the result of obtaining one or more subsequent signals (for example, by sampling) and averaging the integrated signal values of the set of signals, as described above.

Next, in step 502, the controller 126 compares the integrated signal value to an "expected" integrated signal value. The expected integrated signal value may be pre-programmed into the controller 126 (which may include being provided to the controller 126 by an operator at the beginning of a biological fluid separation procedure) or determined by the controller 126. For example, the controller 126 may calculate an expected integrated signal value that is based on the voltage and pulse width of a reference or calibration signal received by the controller 126 during a priming or calibration stage of the procedure, such as the above-described "Saline Calibration Signal." The expected integrated signal value may be calculated according to any suitable approach, though it may be advantageous for the same approach to be employed for calculating both the expected integrated signal value and the integrated signal value for the signal from the light detector 124 being analyzed during the biological fluid separation procedure. The expected integrated signal value may be equal to the integrated signal value of the reference or calibration signal or to a predetermined percentage of the integrated signal value of such a signal (e.g., with the expected integrated signal value being set to 75% of the integrated signal value of the reference or calibration signal).

The next step of the procedure depends on the comparison executed in step 502. When the integrated signal value of the signal received by the controller 126 is greater than or equal to the expected integrated signal value, the controller 126 proceeds to step 504 in which it has determined that there is no need for an adjustment to either the intensity of the light emitted by the light source 122 or the amplification of the signal emitted by the light detector 124 and makes no such adjustment. From there, the controller 126 may either return to step 500 (if the process is to be repeated for a subsequent signal) or may proceed to step 506 in which the signal assessment procedure is ended (if the controller 126 is programmed to check only once during a biological fluid separation procedure whether a dynamic adjustment is required or is at least programmed to not automatically repeat the procedure of Fig. 18 for successive signals).

On the other hand, when the integrated signal value of the signal received by the controller 126 is less than the expected integrated signal value, the controller 126 proceeds to step 508 in which it has determined that a dynamic adjustment (increment) of the intensity of light emitted by the light source 122 and/or the amplification of the signal emitted by the light detector 124 is required and implements such an adjustment. The exact adjustment that is implemented by the controller 126 in step 508 may take any of a variety of possible forms. For example, the controller 126 may only command the light source 122 to emit light having a greater intensity in step 508, with the magnitude of the change being based on a difference between the integrated signal values compared in step 502. In another embodiment, the controller 126 may only command the light detector 124 (which may include commanding an amplification component or module of the light detector 124) to increase the amplification of the signals being generated by the light detector 124, again with the magnitude of the change being based on a difference between the integrated signal values compared in step 502. In yet another embodiment, the controller 126 may command both the light source 122 to emit light having a greater intensity and the light detector 124 to increase the amplification of the signals being generated by the light detector 124, with both changes being informed by the difference between the integrated signal values compared in step 502.

After making a dynamic adjustment in step 508, the controller 126 may either return to step 500 (if the process is to be repeated for a subsequent signal) or may proceed to step 506 in which the signal assessment procedure is ended (if the controller 126 is programmed to check only once during a biological fluid separation procedure whether a dynamic adjustment is required or is at least programmed to not automatically repeat the procedure of Fig. 18 for successive signals).

In one embodiment, the controller 126 may be programmed to first adjust the signal amplification in step 508 before adjusting the light intensity, based on the presumption that the light source 122 is operating properly and that a low integrated signal value is due to an irregularity in the biological fluid (e.g., if separated plasma is lipemic). If the controller 126 then repeats the process of Fig. 18 for a subsequent signal and finds in step 502 that the adjustment to the signal amplification has not been effective to increase the integrated signal value of the subsequent signal to at least equal the expected value, it may proceed to command the light source 122 to increase the light intensity in step 508 to see whether such an adjustment is more effective in increasing the integrated signal value of later signals.

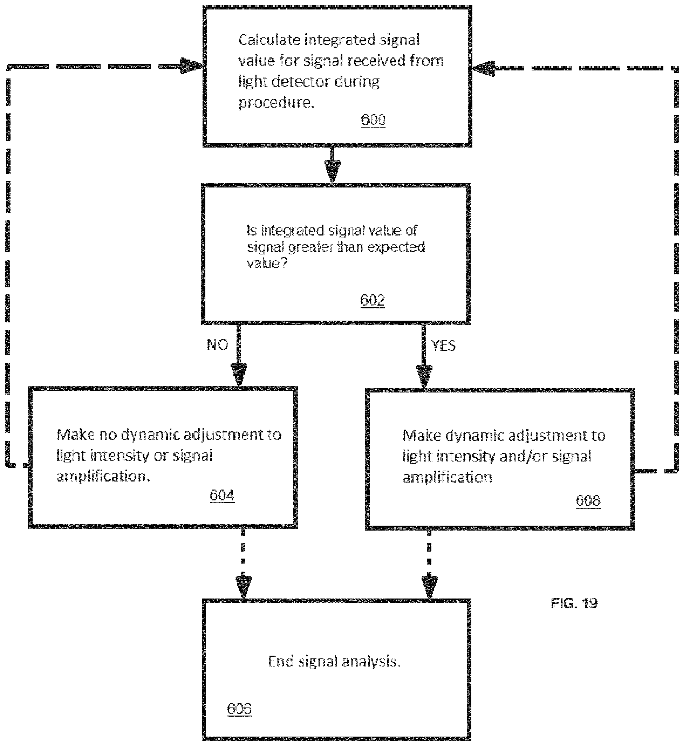

Fig. 19 illustrates a variation of the algorithm of Fig. 18, with the controller determining in step 602 whether the integrated signal value (as calculated in step 600) is greater than an expected value, rather than determining whether the integrated signal value is less than an expected value. When the integrated signal value of the signal received by the controller 126 is not greater than the expected integrated signal value, the controller 126 proceeds to step 604 in which it has determined that there is no need for an adjustment to either the intensity of the light emitted by the light source 122 or the amplification of the signal emitted by the light detector 124 and makes no such adjustment. From there, the controller 126 may either return to step 600 (if the process is to be repeated for a subsequent signal) or may proceed to step 606 in which the signal assessment procedure is ended (if the controller 126 is programmed to check only once during a biological fluid separation procedure whether a dynamic adjustment is required or is at least programmed to not automatically repeat the procedure of Fig. 19 for successive signals).

On the other hand, when the integrated signal value of the signal received by the controller 126 is greater than the expected integrated signal value, the controller 126 proceeds to step 608 in which it has determined that a dynamic adjustment (decrement) of the intensity of light emitted by the light source 122 and/or the amplification of the signal emitted by the light detector 124 is required and implements such an adjustment. The exact adjustment that is implemented by the controller 126 in step 608 may take any of a variety of possible forms. For example, the controller 126 may only command the light source 122 to emit light having a lesser intensity in step 608, with the magnitude of the change being based on a difference between the integrated signal values compared in step 602. In another embodiment, the controller 126 may only command the light detector 124 (which may include commanding an amplification component or module of the light detector 124) to decrease the amplification of the signals being generated by the light detector 124, again with the magnitude of the change being based on a difference between the integrated signal values compared in step 602. In yet another embodiment, the controller 126 may command both the light source 122 to emit light having a lesser intensity and the light detector 124 to decrease the amplification of the signals being generated by the light detector 124, with both changes being informed by the difference between the integrated signal values compared in step 602.

After making a dynamic adjustment (decrement) in step 608, the controller 126 may either return to step 600 (if the process is to be repeated for a subsequent signal) or may proceed to step 606 in which the signal assessment procedure is ended (if the controller 126 is programmed to check only once during a biological fluid separation procedure whether a dynamic adjustment is required or is at least programmed to not automatically repeat the procedure of Fig. 19 for successive signals).

In one embodiment, the controller 126 may be programmed to first adjust the signal amplification in step 608 before adjusting the light intensity, based on the presumption that the light source 122 is operating properly and that a high integrated signal value is due to variations in the signal compared to the baseline (e.g., contamination of a platelet product with white and/or red blood cells). If the controller 126 then repeats the process of Fig. 19 for a subsequent signal and finds in step 602 that the adjustment to the signal amplification has not been effective to decrease the integrated signal value of the subsequent signal to at least equal the expected value, it may proceed to command the light source 122 to decrease the light intensity in step 608 to see whether such an adjustment is more effective in decreasing the integrated signal value of later signals.

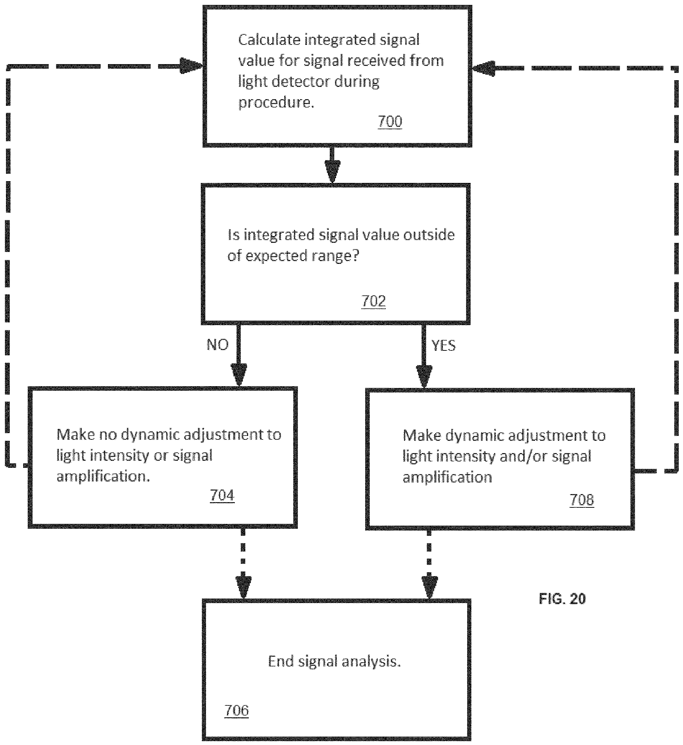

The procedure of Fig. 20 is similar to the procedures of Figs. 18 and 19 but, after the controller 126 calculates an integrated signal value for one or more signals (step 700), it compares the integrated signal value of the signal(s) to an "expected" integrated signal value range (per step 702), rather than comparing the integrated signal value of the signal to a single expected integrated signal value (as in step 502 of Fig. 18 and step 602 in Fig. 19). As explained, the integrated signal value compared to the expected value range may be the result of one or more initial or preliminary steps, with the integrated signal value being, for example, the median integrated signal value across a portion of the signal or of the entire recorded pulse widths, or an average integrated signal value of the signal during the pulse widths. The expected value range may be pre-programmed into the controller 126 (which may include being provided to the controller 126 by an operator at the beginning of a biological fluid separation procedure) or determined by the controller 126. For example, the controller 126 may calculate an expected integrated signal value that is based on the integrated signal value of a reference or calibration signal received by the controller 126 during a priming or calibration stage of the procedure, such as the above-described "Saline Calibration Signal." The expected value range may be based on selected percentages of the integrated signal value of the reference or calibration signal (e.g., with the expected integrated signal value range being set to 75 - 95% of the integrated signal value of the reference or calibration signal).

The next step of the procedure depends on the comparison executed in step 702. When the integrated signal value of the signal received by the controller 126 is within the expected integrated signal value range, the controller 126 proceeds to step 704 in which it has determined that there is no need for an adjustment to either the intensity of the light emitted by the light source 122 or the amplification of the signal emitted by the light detector 124 and makes no such adjustment. From there, the controller 126 may either return to step 700 (if the process is to be repeated for a subsequent signal) or may proceed to step 706 in which the signal assessment procedure is ended (if the controller 126 is programmed to check only once during a biological fluid separation procedure whether a dynamic adjustment is required or is at least programmed to not automatically repeat the procedure of Fig. 20 for successive signals).

On the other hand, when the integrated signal value of the signal received by the controller 126 is outside of the expected range, the controller 126 proceeds to step 708 in which it has determined that a dynamic adjustment of the intensity of light emitted by the light source 122 and/or the amplification of the signal emitted by the light detector 124 is required and implements such an adjustment. The exact adjustment that is implemented by the controller 126 in step 708 may take any of a variety of possible forms. For example, the controller 126 may only command the light source 122 to emit light having a greater intensity (when the integrated signal value is below the expected range) or a lesser intensity (when the integrated signal value is above the expected range) in step 708, with the magnitude of the change being based on a difference between the integrated signal values compared in step 702. In another embodiment, the controller 126 may only command the light detector 124 (which may include commanding an amplification component or module of the light detector 124) to increase the amplification of the signals being generated by the light detector 124 (when the integrated signal value is below the expected range) or to decrease the signal amplification (when the integrated signal value is above the expected range), again with the magnitude of the change being based on a difference between the integrated signal values compared in step 702. In yet another embodiment, the controller 126 may command both the light source 122 to emit light having a different intensity and the light detector 124 to adjust the signal amplification, with both changes being informed by the difference between the integrated signal values compared in step 702.

After making a dynamic adjustment in step 708, the controller 126 may either return to step 700 (if the process is to be repeated for a subsequent signal) or may proceed to step 706 in which the signal assessment procedure is ended (if the controller 126 is programmed to check only once during a biological fluid separation procedure whether a dynamic adjustment is required or is at least programmed to not automatically repeat the procedure of Fig. 20 for successive signals).

In one embodiment, when the integrated signal value is below the expected range, the controller 126 may be programmed to first increase the signal amplification in step 708 before adjusting the light intensity, based on the presumption that the light source 122 is operating properly and that a low integrated signal value is due to an irregularity in the biological fluid (e.g., if separated plasma is lipemic). If the controller 126 then repeats the process of Fig. 20 for a subsequent signal and finds in step 702 that the adjustment to the signal amplification has not been effective to increase the integrated signal value of the subsequent signal so as to bring it within the expected range, it may proceed to command the light source 122 to increase the light intensity in step 708 to see whether such an adjustment is more effective in increasing the integrated signal value of later signals.

Similarly, when the integrated signal value is above the expected range, the controller may be programmed to first decrease the signal amplification, based on the presumption that the default or initial intensity of the light from the light source 122 should not result in a signal having an integrated signal value that is greater than the expected range. If the controller 126 then repeats the process of Fig. 20 for a subsequent signal and finds in step 702 that the adjustment to the signal amplification has not been effective to decrease the integrated signal value of the subsequent signal so as to bring it within the expected range, it may proceed to command the light source 122 to decrease the light intensity in step 708 to see whether such an adjustment is more effective in decreasing the integrated signal value of later signals.

As alluded to above, regardless of the version of the signal analysis protocol that is implemented by the controller 126, the controller 126 may be programmed to execute the protocol once or multiple times during a single iteration of a biological fluid separation procedure. When the controller 126 is programmed to execute the protocol only once, the conditions leading to the controller 126 executing the protocol may vary without departing from the scope of the present disclosure. For example, the controller 126 may be programmed to only execute the protocol when a predetermined volume of biological fluid has been separated during a procedure. In another embodiment, the controller 126 may be programmed to only execute the protocol when a predetermined amount of time has elapsed since the beginning of a procedure. In yet another embodiment, the controller 126 may be programmed to only execute the protocol when a certain procedural event or stage has taken place.

When the controller 126 is programmed to execute the protocol multiple times during a single iteration of a biological fluid separation procedure, it may be programmed to execute the protocol for each signal received from the light detector 124 by the controller 126 or for fewer than all of the signals. For example, the controller 126 may be programmed to execute the protocol once during each stage of a multi-stage procedure or to execute the protocol once every predetermined interval of time (e.g., once every minute). Executing the protocol multiple times during a single iteration of a biological fluid separation procedure may be advantageous to the extent that the results of each execution of the protocol may be stored by the controller 126 and used to assess the status of the centrifuge 102 and/or of the separation chamber 104, 104a. For example, in one embodiment, the controller 126 may be programmed to calculate the number of times that a dynamic adjustment to light intensity and/or signal amplification has been required during a biological fluid separation procedure and, upon determining that the calculated number is at least equal to a maximum number, generate an alert indicating that the centrifuge 102 and/or the separation chamber 104, 104a may be experiencing an irregularity.

In another embodiment, the controller 126 may be programmed to determine a time period during which dynamic adjustment to light intensity and/or signal amplification has been required and, upon determining that the calculated time period is at least equal to a maximum duration, generate an alert indicating that the centrifuge 102 and/or the separation chamber 104, 104a may be experiencing an irregularity. For example, when adjustments implemented over the course of one minute have not been sufficient to produce an acceptable signal, the controller 126 may generate an alert indicating a possible irregularity.

In yet another embodiment, the controller 126 may be programmed to determine the time elapsed after a predetermined event before dynamic adjustment to light intensity and/or signal amplification is required and, upon determining that the calculated time period is not at least equal to a minimum duration, generate an alert indicating that the centrifuge 102 and/or the separation chamber 104, 104a may be experiencing an irregularity. For example, if an adjustment is required too soon after the beginning of a procedure or after a previous adjustment to light intensity and/or signal amplification, the controller 126 may generate an alert indicating a possible irregularity.

When the controller 126 has been programmed to generate an alert, it may be further programmed to provide additional information as to the nature of the irregularity that the centrifuge 102 and/or the separation chamber 104 may be experiencing. For example, the controller 126 may be programmed to recognize a trend in the voltage or integrated signal value of a series of signals as being indicative of a light source 122 that is about to fail, such that an alert generated by the controller 126 may include a suggestion to replace the light source 122. Similar programming (which may include enabling the controller 126 to employ trending and/or outlier analysis techniques) may allow the controller 126 to determine that sinks or voids are present in a ramped surface or that the ramped surface is experiencing crazing or cracking or to diagnose any of a number of other possible irregularities. Additionally, the controller 126 may be programmed to employ machine learning techniques to allow its diagnostic proficiency to improve over time.

The controller 126 may also be programmed to execute the protocol upon the occurrence of certain events that may not occur during a biological fluid separation procedure. For example, the controller 126 may be programmed to execute a signal assessment when there has been a spillover during a procedure, when a change in the blood processing rate of the separation procedure occurs, when a procedure has been paused or stopped, when there has been an alert (different from an alert generated by the controller 126 upon diagnosing a possible irregularity, as described above), or when a spin-down of the centrifuge 102 has occurred.

It will be seen that the techniques described herein help to address possible inconsistency of the overall light intensity of an optical sensor assembly and refine the system with a specific software design, without overhauling the entire hardware design. Dynamically tuning the light intensity at the light source and/or the optical signal amplitude at the amplification circuitry during a biological fluid separation procedure improves the ability of the assembly to accurately maintain the targeted interface location and more reliably results in greater efficiency in processing and a higher quality product than could be expected when conventional techniques are employed.

### Aspects

Aspect 1. An optical sensor assembly of a biological fluid separation system including a centrifuge configured to receive a separation chamber in which a biological fluid is separated into at least two separated components, the optical sensor assembly comprising: a light source configured to emit light having a first intensity toward the separation chamber, with at least a portion of the light exiting the separation chamber as transmitted light; a light detector configured to receive at least a portion of the transmitted light as received light and to transmit a signal having a voltage and a pulse width, wherein the voltage is based at least in part on a second intensity of the received light; and a controller programmed to receive the signal from the light detector and determine a location of an interface between two of the at least two separated components within the separation chamber based at least in part on the signal, wherein the controller is further programmed to control the light source to dynamically adjust the first intensity during a biological fluid separation procedure and/or to control the light detector to dynamically adjust an amplification of the signal during the biological fluid separation procedure.

Aspect 2. The optical sensor assembly of Aspect 1, wherein the controller is programmed to control the light source to dynamically adjust the first intensity during the biological fluid separation procedure and/or to control the light detector to dynamically adjust an amplification of the signal during the biological fluid separation procedure based at least in part on the voltage of the signal.

Aspect 3. The optical sensor assembly of Aspect 2, wherein the controller is programmed to compare the voltage to an expected voltage, dynamically adjust the first intensity and/or the amplification when the voltage is different from the expected voltage, not dynamically adjust the first intensity when the voltage is equal to the expected voltage, and not dynamically adjust the amplification when the voltage is equal to the expected voltage.

Aspect 4. The optical sensor assembly of Aspect 2, wherein the controller is programmed to compare the voltage to an expected voltage range, dynamically adjust the first intensity and/or the amplification when the voltage is outside of the expected voltage range, not dynamically adjust the first intensity when the voltage is within the expected voltage range, and not dynamically adjust the amplification when the voltage is within the expected voltage range.

Aspect 5. The optical sensor assembly of Aspect 1, wherein the controller is programmed to control the light source to dynamically adjust the first intensity during the biological fluid separation procedure and/or to control the light detector to dynamically adjust the amplification of the signal during the biological fluid separation procedure based at least in part on the voltage and the pulse width of the signal.

Aspect 6. The optical sensor assembly of Aspect 5, wherein the controller is programmed to calculate an integrated signal value, compare the integrated signal value to an expected integrated signal value, dynamically adjust the first intensity and/or the amplification when the integrated signal value is different from the expected integrated signal value, not dynamically adjust the first intensity when the integrated signal value is equal to the expected integrated signal value, and not dynamically adjust the amplification when the integrated signal value is equal to the expected integrated signal value.

Aspect 7. The optical sensor assembly of Aspect 5, wherein the controller is programmed to calculate an integrated signal value, compare the integrated signal value to an expected integrated signal value range, dynamically adjust the first intensity and/or the amplification when the integrated signal value is outside of the expected integrated signal value range, not dynamically adjust the first intensity when the integrated signal value is within the expected integrated signal value range, and not dynamically adjust the amplification when the integrated signal value is within the expected integrated signal value range.

Aspect 8. The optical sensor assembly of any one of the preceding Aspects, wherein the controller is programmed to determine whether to dynamically adjust the first intensity and/or the amplification when a predetermined volume of biological fluid has been separated during the biological fluid separation procedure.

Aspect 9. The optical sensor assembly of any one of Aspects 1-7, wherein the controller is programmed to determine whether to dynamically adjust the first intensity and/or the amplification when a predetermined amount of time has elapsed during the biological fluid separation procedure.

Aspect 10. The optical sensor assembly of any one of Aspects 1-7, wherein the controller is programmed to determine whether to dynamically adjust the first intensity and/or the amplification when there has been a spillover during the biological fluid separation procedure.

Aspect 11. The optical sensor assembly of any one of Aspects 1-7, wherein the controller is programmed to determine whether to dynamically adjust the first intensity and/or the amplification when there has been a change in a rate at which the biological fluid is being processed during the biological fluid separation procedure.

Aspect 12. The optical sensor assembly of any one of Aspects 1-7, wherein the controller is programmed to determine whether to dynamically adjust the first intensity and/or the amplification when the biological fluid separation procedure has been paused or stopped.

Aspect 13. The optical sensor assembly of any one of Aspects 1-7, wherein the controller is programmed to determine whether to dynamically adjust the first intensity and/or the amplification when there has been an alert during the biological fluid separation procedure.

Aspect 14. The optical sensor assembly of any one of Aspects 1-7, wherein the controller is programmed to determine whether to dynamically adjust the first intensity and/or the amplification when a spin-down of the centrifuge has occurred during the biological fluid separation procedure.

Aspect 15. The optical sensor assembly of any one of Aspects 1-7, wherein the controller is programmed to repeatedly determine whether dynamic adjustment of the first intensity and/or the amplification is required during the biological fluid separation procedure, determine a number of times that the first intensity and/or the amplification require dynamic adjustment, compare said number of times to a maximum number, and generate an alert when said number of times is equal to or greater than the maximum number.

Aspect 16. The optical sensor assembly of any one of Aspects 1-7, wherein the controller is programmed to repeatedly determine whether dynamic adjustment of the first intensity and/or the amplification is required during the biological fluid separation procedure, determine a time period during which the first intensity and/or the amplification require dynamic adjustment, compare said time period to a maximum duration, and generate an alert when said time period is equal to or greater than the maximum duration.

Aspect 17. The optical sensor assembly of any one of Aspects 1-7, wherein the controller is programmed to determine when dynamic adjustment of the first intensity and/or the amplification is first required during the biological fluid separation procedure, determine a time period that has elapsed between the beginning of the procedure and the first dynamic adjustment of the first intensity and/or the amplification, compare the time period that has elapsed to a minimum duration, and generate an alert when the time period that has elapsed is equal to or less than the minimum duration.

Aspect 18. The optical sensor assembly of any one of Aspects 1-7, wherein the controller is programmed to repeatedly determine whether dynamic adjustment of the first intensity and/or the amplification is required during the biological fluid separation procedure, determine a time period that has elapsed between a previous adjustment to the first intensity and/or the amplification and a current adjustment to the first intensity and/or the amplification, compare the time period that has elapsed to a minimum duration, and generate an alert when the time period that has elapsed is equal to or less than the minimum duration.

Aspect 19. The optical sensor assembly of any one of the preceding Aspects, wherein the controller is programmed to repeatedly determine whether dynamic adjustment of the first intensity and/or the amplification is required during the biological fluid separation procedure, and employ a trending and/or outlier analysis technique during the biological fluid separation procedure to determine whether there is an irregularity in the configuration of the separation chamber based on one or more dynamic adjustments made to the first intensity and/or the amplification during the biological fluid separation procedure.

Aspect 20. The optical sensor assembly of any one of the preceding Aspects, wherein the controller is programmed to repeatedly determine whether dynamic adjustment of the first intensity and/or the amplification is required during the biological fluid separation procedure, and employ a trending and/or outlier analysis technique during the biological fluid separation procedure to determine whether there is an irregularity in the configuration and/or operation of the centrifuge, the light source, and/or the light detector based on one or more dynamic adjustments made to the first intensity and/or the amplification during the biological fluid separation procedure.

Aspect 21. The optical sensor assembly of any one of the preceding Aspects, wherein the light source is configured to be rotated with the centrifuge during the biological fluid separation procedure.

Aspect 22. The optical sensor assembly of any one of Aspects 1-20, wherein the light source is configured to be stationary during the biological fluid separation procedure.

Aspect 23. The optical sensor assembly of any one of Aspects 1-20, wherein the light detector is oriented to receive said received light in a direction generally perpendicular to a direction in which the light is emitted by the light source.

It will be understood that the embodiments described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. An optical sensor assembly of a biological fluid separation system including a centrifuge configured to receive a separation chamber in which a biological fluid is separated into at least two separated components, the optical sensor assembly comprising:
a light source configured to emit light having a first intensity toward the separation chamber, with at least a portion of the light exiting the separation chamber as transmitted light;
a light detector configured to receive at least a portion of the transmitted light as received light and to transmit a signal having a voltage and a pulse width, wherein the voltage is based at least in part on a second intensity of the received light; and
a controller programmed to receive the signal from the light detector and determine a location of an interface between two of the at least two separated components within the separation chamber based at least in part on the signal, wherein the controller is further programmed to control the light source to dynamically adjust the first intensity during a biological fluid separation procedure and/or to control the light detector to dynamically adjust an amplification of the signal during the biological fluid separation procedure.

2. The optical sensor assembly of claim 1, wherein the controller is programmed to control the light source to dynamically adjust the first intensity during the biological fluid separation procedure and/or to control the light detector to dynamically adjust an amplification of the signal during the biological fluid separation procedure based at least in part on the voltage of the signal.

3. The optical sensor assembly of claim 2, wherein the controller is programmed to
compare the voltage to an expected voltage,
dynamically adjust the first intensity and/or the amplification when the voltage is different from the expected voltage,
not dynamically adjust the first intensity when the voltage is equal to the expected voltage, and
not dynamically adjust the amplification when the voltage is equal to the expected voltage.

4. The optical sensor assembly of claim 2, wherein the controller is programmed to
compare the voltage to an expected voltage range,
dynamically adjust the first intensity and/or the amplification when the voltage is outside of the expected voltage range,
not dynamically adjust the first intensity when the voltage is within the expected voltage range, and
not dynamically adjust the amplification when the voltage is within the expected voltage range.

5. The optical sensor assembly of claim 1, wherein the controller is programmed to control the light source to dynamically adjust the first intensity during the biological fluid separation procedure and/or to control the light detector to dynamically adjust the amplification of the signal during the biological fluid separation procedure based at least in part on the voltage and the pulse width of the signal.

6. The optical sensor assembly of claim 5, wherein the controller is programmed to
calculate an integrated signal value,
compare the integrated signal value to an expected integrated signal value,
dynamically adjust the first intensity and/or the amplification when the integrated signal value is different from the expected integrated signal value,
not dynamically adjust the first intensity when the integrated signal value is equal to the expected integrated signal value, and
not dynamically adjust the amplification when the integrated signal value is equal to the expected integrated signal value.

7. The optical sensor assembly of claim 5, wherein the controller is programmed to
calculate an integrated signal value,
compare the integrated signal value to an expected integrated signal value range,
dynamically adjust the first intensity and/or the amplification when the integrated signal value is outside of the expected integrated signal value range,
not dynamically adjust the first intensity when the integrated signal value is within the expected integrated signal value range, and
not dynamically adjust the amplification when the integrated signal value is within the expected integrated signal value range.

8. The optical sensor assembly of any one of the preceding claims, wherein the controller is programmed to determine whether to dynamically adjust the first intensity and/or the amplification when a predetermined volume of biological fluid has been separated during the biological fluid separation procedure, or when a predetermined amount of time has elapsed during the biological fluid separation procedure, or when there has been a spillover during the biological fluid separation procedure, or when there has been a change in a rate at which the biological fluid is being processed during the biological fluid separation procedure, or when the biological fluid separation procedure has been paused or stopped, or when there has been an alert during the biological fluid separation procedure, or when a spin-down of the centrifuge has occurred during the biological fluid separation procedure.

9. The optical sensor assembly of any one of claims 1-7, wherein the controller is programmed to
repeatedly determine whether dynamic adjustment of the first intensity and/or the amplification is required during the biological fluid separation procedure,
determine a number of times that the first intensity and/or the amplification require dynamic adjustment,
compare said number of times to a maximum number, and
generate an alert when said number of times is equal to or greater than the maximum number.

10. The optical sensor assembly of any one of claims 1-7, wherein the controller is programmed to
repeatedly determine whether dynamic adjustment of the first intensity and/or the amplification is required during the biological fluid separation procedure,
determine a time period during which the first intensity and/or the amplification require dynamic adjustment,
compare said time period to a maximum duration, and
generate an alert when said time period is equal to or greater than the maximum duration.

11. The optical sensor assembly of any one of claims 1-7, wherein the controller is programmed to
determine when dynamic adjustment of the first intensity and/or the amplification is first required during the biological fluid separation procedure,
determine a time period that has elapsed between the beginning of the procedure and the first dynamic adjustment of the first intensity and/or the amplification,
compare the time period that has elapsed to a minimum duration, and
generate an alert when the time period that has elapsed is equal to or less than the minimum duration.

12. The optical sensor assembly of any one of claims 1-7, wherein the controller is programmed to
repeatedly determine whether dynamic adjustment of the first intensity and/or the amplification is required during the biological fluid separation procedure,
determine a time period that has elapsed between a previous adjustment to the first intensity and/or the amplification and a current adjustment to the first intensity and/or the amplification,
compare the time period that has elapsed to a minimum duration, and
generate an alert when the time period that has elapsed is equal to or less than the minimum duration.

13. The optical sensor assembly of any one of the preceding claims, wherein the controller is programmed to
repeatedly determine whether dynamic adjustment of the first intensity and/or the amplification is required during the biological fluid separation procedure, and
employ a trending and/or outlier analysis technique during the biological fluid separation procedure to determine whether there is an irregularity in the configuration of the separation chamber based on one or more dynamic adjustments made to the first intensity and/or the amplification during the biological fluid separation procedure.

14. The optical sensor assembly of any one of the preceding claims, wherein the controller is programmed to
repeatedly determine whether dynamic adjustment of the first intensity and/or the amplification is required during the biological fluid separation procedure, and
employ a trending and/or outlier analysis technique during the biological fluid separation procedure to determine whether there is an irregularity in the configuration and/or operation of the centrifuge, the light source, and/or the light detector based on one or more dynamic adjustments made to the first intensity and/or the amplification during the biological fluid separation procedure.

15. The optical sensor assembly of any one of the preceding claims, wherein the light source is configured to be rotated with the centrifuge during the biological fluid separation procedure, or wherein the light source is configured to be stationary during the biological fluid separation procedure

16. The optical sensor assembly of any one of claims 1-15, wherein the light detector is oriented to receive said received light in a direction generally perpendicular to a direction in which the light is emitted by the light source.
